# EUROPEAN PATENT APPLICATION

(11) **EP 2 529 665 A1**
(43) Date of publication of application: **05.12.2012**
(21) Application number: 11450069.7
(22) Date of filing: 30.05.2011
(51) Int. Cl.: A61B 8/08, A61B 5/107, G06T 7/00

(54) **System and method of determining thickness of body tissue**

(71) Applicant: University of Graz, 8010 Graz (AT); Medical University of Graz, 8010 Graz (AT)
(72) Inventor: Müller, Wolfram, 8046 Stattegg (AT); Horn, Martin, 8051 Graz (AT); Ahammer, Helmut, 8503 St. Josef (AT)
(74) Representative: Patentanwaltskanzlei Matschnig & Forsthuber OG

(57) **Abstract**

For the determining the thickness of a tissue layer based on an image recorded with an imaging process such as ultrasound imaging (11), an image describing spatial behavior of a measured quantity across at least one section through said tissue layer is recorded (13) and processed in a data processing apparatus, which calculates values of thickness for said image data. For this calculation, a plurality of section lines within the image is used, thickness value candidates for each of the section lines are determined (17), the thickness value candidates are sorted into distance classes (19), of which at least one is selected, and a value of thickness is calculated by averaging (21) the thickness value candidates within the distance class(es) selected. The individual distance values can also be used for analyses of patterns, e.g. subcutaneous fat patterning in the imaged part of the body.

## Description

### Field of the Invention And Description of Prior Art

The invention relates to a system and a method for determining the thickness of a tissue layer based on an image recorded with an imaging process, preferably ultrasound imaging or another medical imaging technique.

The technical and physical principles of ultrasonic imaging are based on the pulse-echo technique. A piezoelectric transducer applies a short ultrasound pulse which travels with the speed of sound (c) in the given tissue; ultrasound echoes are detected and measured with regard to travelling time and intensity. Most diagnostic ultrasound machines use c = 1540 m/s for calculating the distance (d = c T/2, with T being the echo time) from the source to the boundary between two tissues having different acoustic impedances. For 2D imaging (B-mode ultrasound) many ultrasound beams are sent sequentially into the tissue for creating an image in which the brightness of the screen corresponds to the echo intensity in the plane of the scan. Because of diffraction, which limits transverse spatial resolution, only objects larger than or approximately equal to the wavelength used can be imaged. For diagnostic images, usually frequencies between 3 and 22 MHz are used, corresponding to wavelengths in soft tissue of 0.5 to 0.07 mm. Maximum usable frequency is limited because attenuation of sound increases with higher frequencies.

While the invention was developed primarily with regard to ultrasound measurements, it will be clear to the skilled person that the invention may use other types of images for distance measurements as well. For any kind of such images, in particular digital images, the evaluation procedure is similar to the evaluation method for ultrasound images, which is described here in the first instance.

The high accuracy of ultrasound measurements of fat thickness and other tissues in humans are well established. Ultrasound imaging can be applied to thickness measurements of subcutaneous adipose tissue (SAT) and muscle, muscle cross-sectional area, and abdominal depth. The precision of SAT measurements was found to be excellent (A. Bellisari et al., in Int J Obes Relat Metab Disord 1993 17 (8): 475-80, found that the technical error in both intra- and inter-observer studies was less than 0.15 mm at the following sites: suprailiac, paraspinal, sacral, epigastric, mid-thigh; and 0.6 mm at the triceps). Y. Ishida et al., Am J Hum Biol 1992 4: 511-20, found B-mode ultrasound to be a highly reliable method for measurement of both fat and muscle thickness. Ultrasound imaging has also been suggested for visceral fat mass evaluation, T. Abe et al., Appl Human Sci 1995 14 (3): 133-9. Recently, Moore et al., in Rheumatology 2003 42 (12): 1559-63, described an ultrasound approach for precise and accurate measurement of skin thickness using a 22 MHz transducer. In principle, in all tissues and organs of the body which can be penetrated by ultrasound and where reflections occur at transitions to adjacent tissues, distances can be measured and accuracy depends primarily on the choice of sound seed setting for appropriate distance calculation. However, well-known ultrasound imaging artifacts may cause failures.

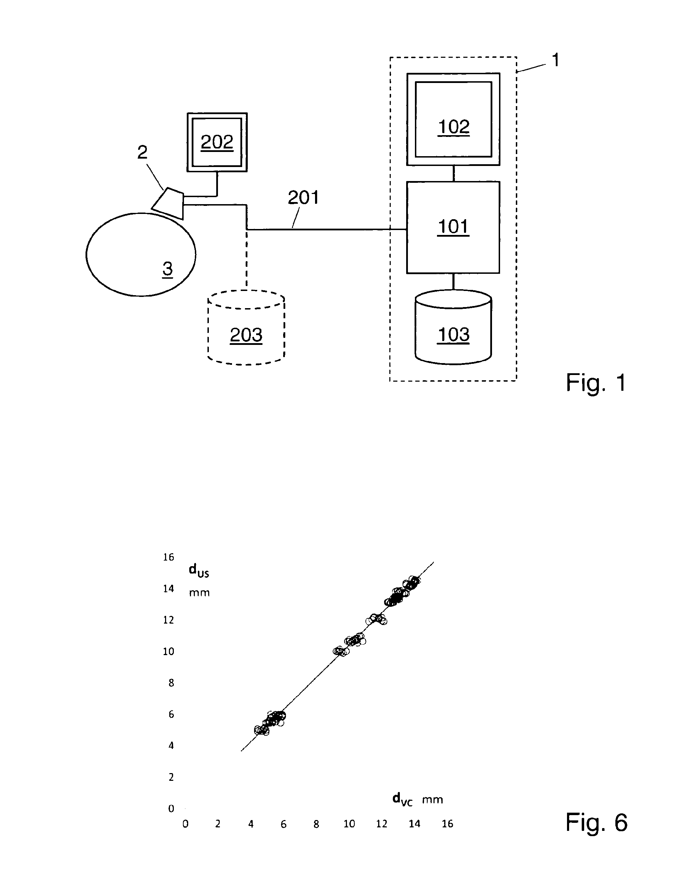

In a recent experiment the inventors compared adipose tissue thickness measurements performed in a dissected swine carcass at 22°C by means of ultrasound to measurements made using a vernier caliper. Ultrasound thickness values and vernier caliper measurements (resolution of 0.01 mm) were highly correlated (r = 0.998; n = 140; *f* = 7.5 MHz). This is also shown in Fig. 6, where the thickness values d_{vc} obtained using the vernier caliper are correlated with the thickness values dus; the thickness values are given in millimeters. The regression coefficient (slope; straight line in Fig. 6) was 0.98 when a standard sound velocity of 1540 m/s was used; a coefficient of 1.00 was obtained with 1510 m/s for distance calculation, indicating a lower speed of sound in fat. The standard error of estimate (SEE) was 0.21 mm.

Studies designed for accurate determination of speed of sound in human adipose tissue at 37°C would further increase the accuracy of this method. Measurement error due to sound speed deviation is proportional to the deviation from the real speed in the tissue (e.g. 6% for a speed deviation of 100 m/s, or 0.6 mm in a 10 mm thick tissue). Accuracy demands beyond the capability of ultrasound are of little relevance because of accuracy limitations due to the tissue's plasticity and uneven tissue contours. It is expected that appropriately applied ultrasound pulse-echo technique is capable of thickness measurements in biological tissues with highest accuracy possible because the accuracy limitations depend primarily on the plasticity and deformability and uneven contours of the tissues. Also in other materials to which ultrasound pulse-echo technique can be applied, ultrasound can be used for highly accurate and precise distance measurements.

Due to the high accuracy of SAT measurements at given sites, ultrasound can be used to calibrate other imaging techniques like MRI or CT. Also, ultrasound could be used for optimizing the image segmentation protocol, which is always a crucial problem in MRI and CT image analysis. For instance, comparative studies of MRI and ultrasound measurements for visceral and subcutaneous fat evaluation were presented by M. Koda et al. in Abdom Imaging 2007 32 (3): 387-92.

Various software packages are available for image analyses and for distance measurements in ultrasound images by means of manually set cursors in the image. Current ultrasound equipment enables measurement of a single distance or of a few distances by means of consecutively set cursors (calipers); determination of larger numbers of distances is very time-consuming with currently available software.

In WO 2006/062867 A2 a method for determining the organ wall mass using a three-dimensional ultrasound recording is disclosed. The wall mass, for instance a bladder weight, is calculated as a product of the bladder surface area, the bladder wall thickness and the bladder wall specific gravity.

There are, however, various problems with the current technology.

Most ultrasound medical imaging devices enable measurement of distance by means of manually positioned cursors at desired sites in the image and by calculating the distance d between 2 cursor positions according to d = c T/2, wherein T denotes the echo time and c is the speed of sound. Measurement of many distances in a given image is time consuming due to the manual cursor positioning necessary and the buttons to be pressed for each measurement.

Another problem concerns the choice of adequate speed of sound used for determination of distances based on pulse-echo time measurements. Current medical imaging devices are not designed to apply various speeds of sound easily for distance determination in a given ultrasound image at different regions of interest (ROI) in order to enable applying appropriate speed of sound (c) in given tissues. For instance, in adipose tissue (fat) the speed of sound is lower than the usually applied value of c = 1540 m/s, as mentioned, and in cartilage tissue c is larger than this value.

Manual cursor setting for distance measurement depends on the visual image and individual assessment of tissue border zones by the investigator and failures may occur due to the subjective human eye's interpretation of the grey values in the transition zones in terms of tissue boundaries. Inter-observer-variability and intra-observer-variability may therefore be high when compared to automatic or computer-assisted approaches.

Furthermore, with current equipment, it is difficult to perform the detection of series of thickness values according to an individual sort of tissue (for example a layer of subcutaneous adipose tissue) or series of distance measurements in several tissues imaged in a single ultrasound image (for example: skin, subcutaneous adipose tissue, muscle) in a precise, accurate and time-saving manner.

Currently, medical ultrasound imaging equipment is not available which combines low cost ultrasound devices with image analysis software enabling highly precise and accurate measurement of series of distances in imaged tissues of the body or in other materials. With many ultrasound machines available today, particular in lower cost models, it is difficult or impossible to set cursors precisely and accurately because of limited resolution of the scroll ball or other computer-human interfaces used.

There is no method for image analysis available which enables highly accurate measurements based on a spatial image of the tissue to be investigated. The expression "spatial image" is used here to denote a two- or three-dimensional image relating to a two-dimensional (such as a section) or three-dimensional region of the human (or animal) body.

### Summary of the Invention

It is an aim of the present invention to provide a reliable, automatized measurement of the thickness of tissues in the human or animal body, preferable of human fat layers. The invention should enable to process a large number (e.g., hundreds) of distances in short time in one or in several regions of, e.g., an ultrasound B-mode image in which speed of sound may vary from one region of interest to the other.

This aim is obtained by a system for processing an image describing spatial behavior of a measured quantity across at least one section through said tissue layer, which comprises a data processing apparatus configured to accept images in form of image data and calculate values of thickness for said image data by
- taking a plurality of distance measurement direction lines (also referred to as "section lines") within the image,
- determining thickness value candidates for each of the section lines,
- sorting the thickness value candidates into distance classes,
- selecting at least one distance class, and
- calculating a value of thickness by averaging the thickness value candidates of the one or more distance classes that were selected in the previous step.

The system may also comprise an imaging equipment, e.g. ultrasound imaging equipment, configured to produce image data relating to an image, which was recorded beforehand at the patient's body, and further configured to communicate said image data to the data processing apparatus.

Likewise the above-mentioned aim is met by a method for determining the thickness of a tissue layer, with the method comprising the above-cited steps.

The invention enables automatic or semi-automatic measurements of series of thickness values (up to several hundred values) from various types of digital images derived from all kinds of medical or other imaging techniques in short time (hundreds of distance measurements from a single image within the time of a second) and with high accuracy.

The invention includes an effective finding of distance classes (intervals) according to layers of various tissues in a given image. The invention also makes use of edge detection and texture classification algorithms which are well-known and available for general applications, but were not, to the knowledge of the inventors, used in the context of tissue thickness measurement.

The investigation uses a digital image evaluation algorithm which interprets image information always in the same pre-defined way (threshold setting, derivation of regions of interest, choice of distance calculation parameters and other settings) and therefore subjective human eye's interpretation failures and resulting distance measurement deviations when performing manual evaluation do not occur and therefore this invention can reduce inter-observer-variability and intra-observer-variability.

The method is well suited for tissue patterning studies, for example subcutaneous fat patterning. The automatic approach combined with the possibility of an inter-active approach ensures high validity of the measurement results because of the advantages of visual control of the automatic image analysis which results from the evaluation algorithm.

In a further development of the invention the step of determining thickness value candidates comprises determining the positions of extreme values of the measured quantity, and calculating the distance between the positions of extreme values thus obtained to obtain said thickness value candidates. before the step of calculating the distance between the positions, an additional step of rejecting those extreme values which are out of a threshold window, keeping only the positions of extreme values within said threshold window, may be included.

Moreover, a calculation of statistical data with regard to the thickness value candidates of the distance class(es) selected may be included.

Furthermore, it is often advantageous to select a region of interest within the image, and the section lines are then taken only within said region of interest (ROI). Otherwise, the entire image will be taken as ROI.

The process may also be performed in an iterative manner. Thus, after the step of selecting at least one distance class, the distance class(es) selected may be used as ROI and the method, starting from one of the steps of taking a plurality of section lines within said region of interest or a subsequent step, is repeated with respect to this ROI.

By virtue of the present invention, particularly in the case of ultrasound image evaluation, very high distance measurement accuracy can be obtained. This distance determination is predominantly limited by the soft tissue's plasticity. Several tissues with differing sound speeds within a given ultrasound image can be analysed by choosing respective regions of interest and by setting appropriate sound speeds for the individual ROI.

The present invention enables highly accurate fat layer thickness measurements by means of ultrasound which is an important diagnostic information and can be obtained from all kinds of medical ultrasound equipment, and more than that, it is applicable in low cost ultrasound imaging devices using transducers with a single (A-mode imaging) or a lower number of crystals (ultrasound beams) because accurate tissue depth measurements do not necessarily need high spatial resolution in a direction transverse to the direction of distance (depth) measurement. It also does not need high time resolution of the image detection equipment and this makes it also possible to apply low cost ultrasound hardware devices for highly accurate distance determinations. With ultrasound equipment becoming available in notebooks and possibly even in mobile phones or other electronic processor-based mass products (when inter-active image evaluation is also desired it has to have a display too), a large number of applications can be expected.

### Brief Description of the Drawings

In the following, the present invention is described in more detail with reference to the drawings, which show:

| | |
|---|---|
| Fig. 1 | a schematic block diagram of a system for carrying out the tissue thickness analysis according to the invention; |
| Fig. 2 | is a flow chart of an automatic tissue thickness analysis, including visual control and manual interaction possibilities; |
| Fig. 3 | shows an exemplary B-mode ultrasound image used for a thickness analysis; |
| Fig. 4 | illustrates the choice of a region of interest within the image of Fig. 3; |
| Fig. 5 | shows a distance analysis for the region of interest selected in Fig. 4; and |
| Fig. 6 | a comparison of measurements of fat layer thickness made by ultrasound and vernier caliper. |

### Detailed Description of the Invention

In the following an exemplary implementation of the invention as an evaluation software is described, which provides for automatic tissue thickness analysis with visual control and manual interaction possibilities starting from an image data file obtained from an imaging method like B-mode ultrasound scan. The software can be implemented on a suitable platform, such as a PC, workstation computer or a handheld computer equipped with a detection device such as an ultrasonic probe, and can be configured for processing of any kind of digital image.

### System Layout

The block diagram of Fig. 1 illustrates one possible embodiment of a system to determine fat layer thickness according to the invention. A data processing apparatus 1, in this case a PC 101 with a display 102 and an associated replaceable storage device 103 for permanent storage and/or archiving of data files, is used as a platform for the evaluating software. The apparatus 1 is connected with an ultrasound imaging device 2. Ultrasound imaging devices able to produce images in form of image data that can be processed externally are well known in prior art. Usually the ultrasound imaging device 2 is provided with a display 202 for ease of use by the practitioner, to better find a suitable location at the patient's body 3 for recording an image that is suitable for the evaluating process. The image data may be transferred directly to the data processing apparatus 1, or via an intermediate storage device 203 (which is, for instance, a hard disk, a USB data stick, or any other suitable storage means. The data connection 201 can be realized using a data line, a wireless connection or, as an alternative to a data communications line, by using a temporary storage component as "data shuttle", such as a USB storage device (not shown) connected alternatingly with the PC 101 and the device 2.

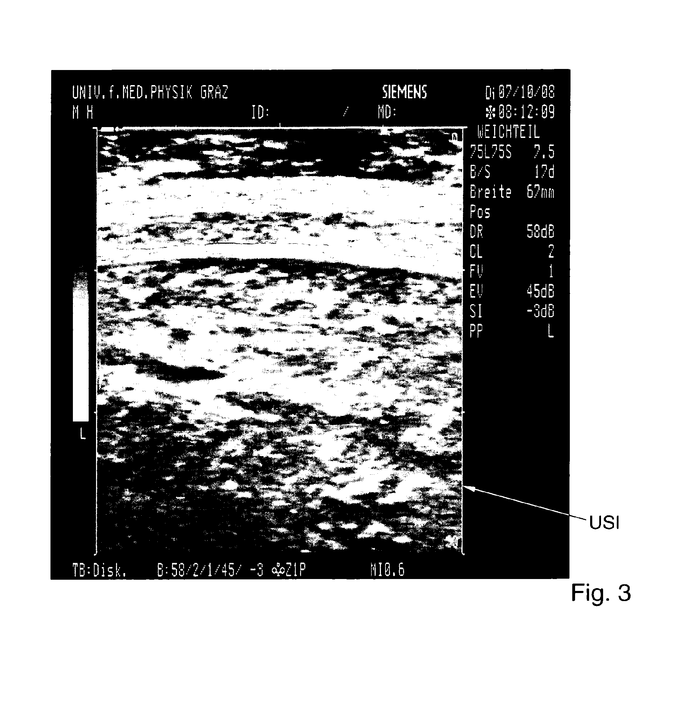

In the image data, an example of which is shown in Fig. 3, grey values (or, depending on the data representation used, colours) will represent real-world physical quantities. At border zones of tissues (structures) the values of the physical quantity (the nature of which depends on the a imaging technique used respectively) and, consequently, the grey values will change accordingly. This can be utilised to identify tissues or organ parts of interest. In particular, changes of grey values as a function of position for a tissue can be typical for a region of interest. These grey value changes may be changes of border zones of a tissue or texture patterns in the tissue or characteristic geometrical or anatomical or histological structures. Additionally, typical grey value changes at border zones enable measurements of tissue thicknesses and allow definition of distance classes which define regions of interest.

As already mentioned, the invention includes an automatized selection of region of interest (ROI) and enables automatic or semi-automatic measurements of series of thickness values (up to several hundred values) from various types of digital images derived from various kinds of medical or other imaging techniques, within short time (hundreds of distance measurements from a single image) and with high accuracy. The evaluation software is described below for ultrasound images; but the operating principles are straightforward to be used with any other kind of digital images.

### Overview of Algorithm

The method and software program according to the invention will also be called image evaluation caliper in accordance with the imaging technique used; for instance, it will be called 'ultrasound caliper' when based on ultrasound measurements, and likewise for the other imaging techniques.

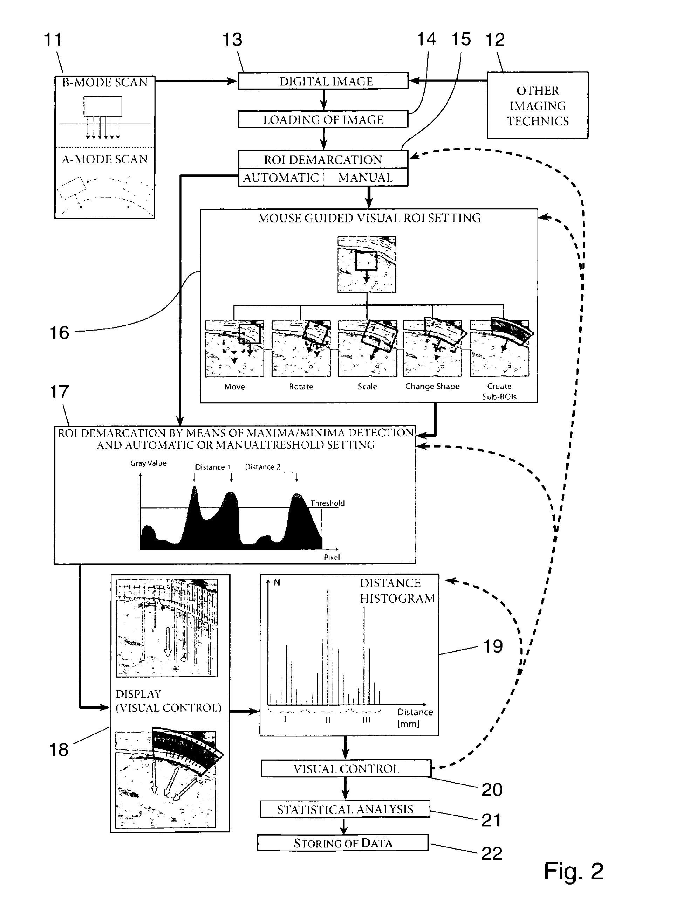

Fig. 2 shows a flow chart illustrating the algorithm underlying the evaluation software according to the invention.

In a first step 11, an image is taken from the anatomic region to be examined (image acquisition). The embodiment shown here is based on the use of a B-mode scan picture. In a variant, an A-mode scan may be used as well. Beside an ultrasound measurement ('ultrasound' is often abbreviated as US), another imaging technique 12 can be used as sources for distance analyses as well, such as magnetic resonance imaging (MRI), computer tomography (CT), X-ray imaging, in particular using projected distances or digital radiography, emission tomography (ET) including in particular SPECT (single-photon emission computed tomography) and PET (positron emission tomography), impedance tomography (IT), near-infrared spectroscopy (NIRS), a "sonar" acoustic depth finder designed for multiple depth determinations, or any other imaging techniques which result in digital images.

The image data 13 produced during image acquisition are stored in digital form in one of several common formats, such as jpeg, tiff, a bitmap format such as bmp or png, or a DICOM file. In the present example, a tiff image is used. The size of the image may vary, possibly depending on size limitations imposed by the hardware used.

In step 14 the evaluation program is started and the image data are loaded into the evaluation program. In a variant, the image acquisition may be controlled by the evaluation program as well. Also, variations of the image acquisition and processing parameters provided by the ultrasound equipment (in the example of ultrasound applications) can be used in parallel to control parameter variations in the evaluation program according to the invention in order to optimize the image for distance measurement purposes. If required, landmarks, e.g. marked on the skin surface, may be indicated in the ultrasound image by masking out individual or groups of ultrasound beams which are in well-known distance to the landmarks. Mechanical masks on the transducer surface, appropriately designed for given situations, may be used for this purpose, or alternatively, individual ultrasound beams or groups of ultrasound beams may be blocked out by electronic means (such as by blocking the respective electrical signal transferred to the crystals of the transducer).

The next step 15 starts the selection of a ROI (also referred to as ROI demarcation). The user can choose whether the ROI is to be selected automatically (by a routine within the program) or manually. With an automatic ROI selection, for a given region to be detected, an appropriate algorithm capable of detecting the specific characteristics is used. For example, in case of tissue layers like subcutaneous adipose tissue in which thickness does not change much, the criterion is to define the distance class such that only a distinct variation of distances is accepted. Such criteria can be implemented for each object of interest.

Alternatively or in combination with automatic selection, ROI demarcation may be made manually in a step 16, using a mouse-guided GUI for setting a selected region within the area (or range) of the image, in combination with a visual display on which the image is shown: The setting of ROI position, size, orientation, shape, as well as possible selection of sub-ROI and weighing, is made using well-known methods for selecting a range in a graphic area.

The ROI demarcation is completed in step 17 by a determination of maxima/minima. For this purpose edge detection software protocols for optimally adapted thickness measurements in given tissues may be selected and set. One possibility is the use of morphological operators like Roberts- or -Sobel-Operators or the approach sketched in step 17: The gradient of grey values is derived within the image data, and local minima/maxima or significant changes for a given tissue border zone can be used for defining the end points of a distance to be measured. Furthermore, a pre-set threshold may be used to ensure that extreme values due to local fluctuations and noise are ignored. In many cases, for instance in the case of ultrasound distance measurements in subcutaneous adipose tissue, constant minimal or maximal values can be spread across several pixels; in such cases the edge is selected as the first pixel adjacent to the adipose tissue. For the threshold value a default values can be preset, e.g., 50% or 60% or 70% of the amplitude or dynamic range of the image data.

In step 18, distance values are calculated for a number of lines within the ROI selected, and the measured distances (i.e., layer thickness values) are graphically overlaid with the image represented on the graphical display.

Furthermore, the distance values determined in step 18 are counted and displayed in a distance histogram 19, which displays for each possible distance value (given in mm for instance) the number N of occurrences of the respective distance value.

Based on the histogram 19, distance classes are determined as described below: In the example shown in Fig. 2, distance classes are numbered by roman numerals I, II, III. In case of automatic region of interest definition, various criteria can be used for the selecting the distance class. For instance, for a layer of almost constant thickness a suitable criterion is to use the largest class (i.e., the class having the largest number of detected distances); in case of a wedge-shaped structure a constant gradient is a criterion for finding a distance class suitable for evaluation. Likewise, the criterion can be adapted to any given geometrical structure in an analogous manner.

At this point it is advantageous to allow a visual control of the collected data, and based on the input of the user, to optionally repeat the algorithm re-starting from any of steps 15 to 19.

Furthermore, specific parameters which are characteristic of the measurement can be selected at this stage. In particular, in case of ultrasound images, this will include the selection of the appropriate value(s) of speed of sound according to the imaged tissues from a catalogue of predefined values (in accordance with sound speeds of various types of tissue).

Once the data are prepared to a satisfying extent, a statistical analysis 21 is performed. The results obtained are stored and/or displayed, including statistical parameters such as mean, standard deviation, median, extreme values, skewness, kurtosis. The data may be displayed by any known graphical display mode, such as histograms, bar charts, box plots.

In an optional final step 22, the data, images, and distance overlaid images may be stored and/or exported to a permanent storage device such as a floppy or hard disk. Any suitable format may be used, such as the above-mentioned data formats, chosen in accordance with suitable software and amount of data.

For a position determination of the ultrasound transducer, investigation sites can be determined by the anatomical structures imaged or by anatomical land marks on the body surface. Highly accurate and low cost transducer position allocation (and thus ultrasound image pixel allocation) with respect to given marked points on the body surface (skin) can be obtained by using ultrasound-guided biopsy equipment applying a marker instead of the biopsy needle in order to determine the geometric position of the transducer with respect to anatomical landmarks. For example, for comparisons of subcutaneous fat tissue thickness measurements with skinfold measurements, positions where skinfolds are measured have to be aligned with the ultrasound fat measurements. Such and all similar problems can easily be handled without extra costs by using this additional device which consists just of a marker which fits in the needle holder. A variety of mechanical transducer position marking devices can be built in a similar way.

### Example

Below, a specific example of an evaluation of an ultrasound image is described.

As a starting step, an ultrasound image is taken according to known techniques. In the present example (human calf), shown in Fig. 3, a B-mode ultrasound image, denoted by reference symbol USI, of size 515 × 512 pixels in .TIFF format is created by a Siemens Sonoline PRIMA ultrasound system, using the 7.5L75S linear transducer.

The B-mode image is loaded into an evaluation program implementing the algorithm explained above with reference to Fig. 2. This evaluation program was written as a program code in IDL v6.1 (Research Systems Inc.) and dubbed "ultrasound caliper".

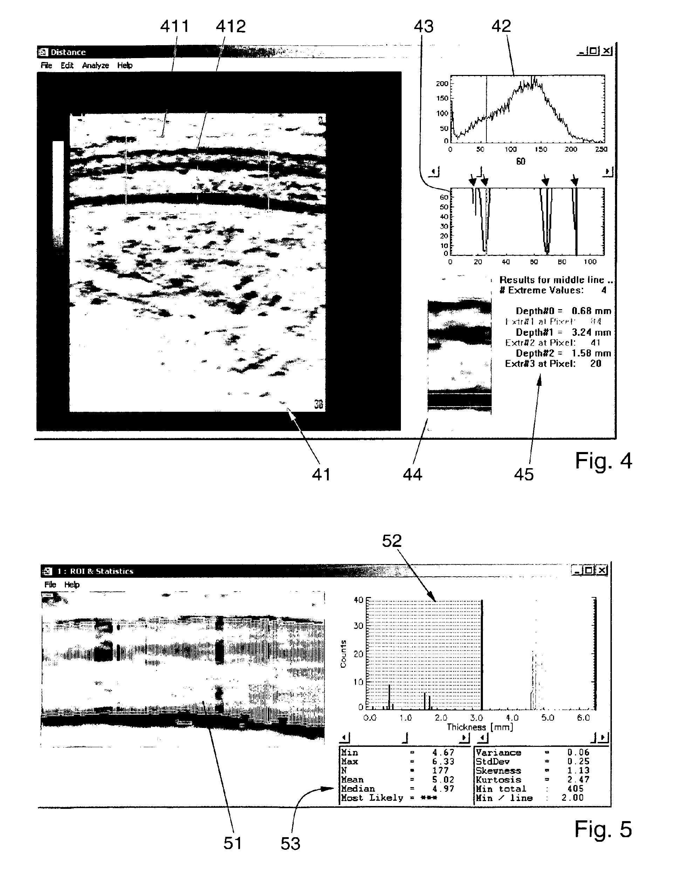

Fig. 4 shows a window 40 of the graphical interface of the mentioned program. The window includes a representation 41 of the ultrasound image USI of Fig. 3, as well as a number of additional data output 42, 43, 44, 45, explained below.

Within the ultrasound image a region of interest (ROI) is selected. In Fig.4 a rectangular frame 411 represents the ROI. The ROI has width of 200 pixels and height of 100 pixels, i.e., comprises 20.000 pixels. As an example for an automatic determination of the ROI, the distances found in the whole image are classified by k-means clustering or another method cluster analysis. An alternative, very simple way for automatic ROI determination is excluding all distance values beyond a predefined range around a predefined absolute distance value.

A grey-value histogram 42 is determined within the ROI and displayed. On the horizontal axis the grey-values are indicated (0 to 256 grey-values); on the vertical axis, the number of grey-values as a function of grey value is shown; the vertical red line represents the grey-value threshold (60 in this case). Local minima (shown with the darkest grey colors in the image 41) are detected only below this cut-off-value. (In the case of an inverted image - light grey shades instead of dark grey shades - maxima above this value would be detected.)

Along "section lines" running vertical in the ROI (or generally, along section lines having uniform orientation within the ROI), a grey-value profile is taken. These lines correspond to the distance measurement direction lines or section lines according to the invention. One vertical section line is highlighted by dashed line 412 in the image 41. For this one highlighted section line 412 the analysis is shown in the right-hand part of Fig. 4: In particular, the corresponding grey-value profile 43 is displayed. On the horizontal axis, the pixels of the line 412 are indicated; on the vertical axis, the corresponding grey-value is shown. Because of the grey-value threshold used here (60 in this case), the vertical axis is limited to the range below the threshold. Detected minima are indicated by vertical lines, which on a color display may be emphasized in different colors for better clarity; in Fig. 4, the position of these vertical lines is marked by small arrows at the upper frame line of the profile 43.

The analysis shown here for one vertical line 412 is repeated by the algorithm for the complete set of vertical lines within the ROI 411. In a more general version, section lines are taken within the image or ROI. The section lines may be oriented in any angle, and the profile analysis is done for a set of parallel lines within the ROI. The angle of orientation can be fixed (default value can be chosen; vertical in this case) or adjusted manually between 0° to 180°. In the specific example discussed here, the simplest approach is chosen, namely, to take the vertical lines of pixels as section lines according to the invention.

Furthermore, an additional image 44 may be included, showing an enhanced display of the area surrounding the highlighted section line 412. Detected minima at the site of the vertical line 412 are indicated by horizontal lines across the image 44. The distances calculated for line 412 between the minima are shown in a table 45 within the display. These distances represent the distance value candidates according to the invention.

If a color graphic display is used, colors may be used to additionally identify each of the maxima/minima within the display fields 43, 44, 45.

Fig. 5 shows a second window to display a distance analysis. Since the ROI has a width of 200 pixels, 200 lines (each of the type as line 412 of Fig. 4) are analysed. The result of the analysis may be shown in a graphical depiction 51 which is an enhanced depiction of the ROI, in which for each section line the calculated distance candidates are highlighted (vertical white line segments). Local minima (here minima = dark grey or black shades) are indicated by small squares. In accordance with the invention, the distances between them are calculated and are highlighted in the graphical depiction 51; if the display allows colors, the distances could also be shown in color code.

In the right-hand part of the window a histogram 52 of all found distances within the ROI is shown, as well as a statistics 53 of the distances determined. The horizontal axis indicates the distance in millimeters; the vertical axis shows the number of calculated distances. The vertical solid black line right to the dotted area represents the lower distance threshold (in this case: 3.25 mm). The upper distance threshold is indicated by the vertical solid black line at the right of the histogram (at 6.5 mm). The dotted area is neglected in further calculation due to threshold setting. This definition of the ROI was done automatically (using preset values). The statistics field 53 shows various characteristic statistical quantities of the set of distances. In this case, only distances between 3.25 mm and 6.5 mm are included (the distance interval evaluated can be set automatically or manually).

### Characteristics of the Invention

The measuring procedure combined with the evaluation software is unique in that it enables an approach for high accuracy, high precision, high validity, and rapid determination of large numbers of distance values (up to several hundred from a single ultrasound image) from all kinds of human or animal tissues or from other structures investigable by means of ultrasound; moreover, the algorithm can be applied to all kinds of images, in particular digital images.

The invention can not only be implemented using ultrasound B-mode imaging; for development of low-cost devices - but independently of lower costs still designed for obtaining high precision and high accuracy measurements - transducers with low numbers of crystals (i.e. low number of ultrasound beams sent into the tissue) or also A-mode technique devices (after appropriate data pre-processing) can be used.

The software includes multiple choices of sound speeds used for pulse-echo interpretation (in terms of distance) in accordance with the speeds of sound in various tissues imaged in a B-mode scan and provides a distance evaluation in ultrasound images based on the respective appropriate speed of sound. The software applies both the advantage of automatic distance analysis of various tissue layers (imaged in a single ultrasound image) and manual analysis and improves validity by enabling cross-checking and correcting of automatic image analyses by means of the investigator's visual control. The algorithm detects many (up to several hundred) distances of a tissue layer within parts of a second and also enables setting of appropriate speeds of sound in various tissues displayed in the ultrasound image.

The ultrasound image analysis in terms of multiple distances measurements by means of conventional ultrasound equipment and the algorithm according to the invention results in highest accuracy possible for soft tissue (in which accuracy is naturally limited by the plasticity of the tissue).

The software enables a highly valid ultrasound image analysis in terms of tissue thickness evaluation with an accuracy as high as theoretically possible, which is only limited by the uneven tissue contours and the plasticity of soft tissues which amount to measurement deviations larger than the accuracy obtainable with ultrasound which is a few times the applied ultrasound wavelength of 0.5 to 0.07 mm, according to the ultrasound frequency which can be chosen for medical imaging between 3 and 22 MHz, depending on the desired resolution and on the depth of the ultrasound investigation in the body.

The measuring procedure uses ultrasound markers (mask on the skin or on the transducer) for determination of anatomical/anthropometrical position. Image analysis software determines series of thickness values in the automatically or manually chosen region of interest (ROI) of the ultrasound cut plane and also gradients of thickness values which can additionally be used for automatic limitation of region of interest (ROI). The software is also capable of limiting the amount of data to a minimum necessary for tissue thickness analysis which is an advantage particularly for low cost applications. Additionally, the algorithm can also be used for echo time data from A-mode scans after appropriate data pre-processing.

In practice, interfaces are often not parallel or contours of tissues are irregular or rough. It is another advantage of the techniques described here, and in particular ultrasound, that many measurements can be made in the vicinity of a given site, even where thickness varies greatly, and mean values can be used instead of single point measurements. Thus, the standard error of measurement can be kept very low by using many measurements in the vicinity of the given site.

The foregoing description of specific embodiments is of exemplary nature only and is not limiting for the present invention. Rather, the invention encompasses all embodiments and implementations which the person skilled in the art can conceive and realize within the scope of the claims. Also, it will be evident to the skilled person that various modifications and additions can be made to the embodiments to realize the invention.

## Claims

1. A system for determining the thickness of a tissue layer based on an image recorded with an imaging process, preferably ultrasound imaging, said image (41) describing spatial behavior of a measured quantity across at least one section through said tissue layer, comprising a data processing apparatus (1) configured to accept images in form of image data and calculate values of thickness for said image data by
- taking a plurality of section lines (412) within the image,
- determining thickness value candidates (45) for each of the section lines (412),
- sorting the thickness value candidates into distance classes (19),
- selecting at least one distance class, and
- calculating a value of thickness by averaging the thickness value candidates of the distance class(es) selected.

2. The system of claim 1, further comprising an imaging equipment (2), preferably ultrasound imaging equipment, configured to produce image data relating to an image recorded with said imaging equipment and further configured to communicate said image data to the data processing apparatus.

3. A method for determining the thickness of a tissue layer based on an image recorded with an imaging process, preferably ultrasound imaging, said image (41) describing spatial behavior of a measured quantity across at least one section through said tissue layer, the method comprising the following steps:
- taking a plurality of section lines (412) within the image,
- determining thickness value candidates (45) for each of the section lines (412),
- sorting the thickness value candidates into distance classes (19),
- selecting at least one distance class, and
- calculating a value of thickness by averaging the thickness value candidates of the distance class(es) selected.

4. The method of claim 3, wherein the step of determining thickness value candidates comprises:
- determining the positions of extreme values of the measured quantity, and
- calculating the distance between the positions of extremal values thus obtained to obtain said thickness value candidates.

5. The method of claim 4, further comprising a step of
- rejecting those extreme values which are out of a threshold window, keeping only the positions of extreme values within said threshold window,
before the step of calculating the distance between the positions thus obtained.

6. The method of any one of claims 3 to 5, further comprising the calculation of statistical data with regard to the thickness value candidates of the distance class(es) selected.

7. The method of any one of claims 3 to 6, further comprising the step of
- selecting a region of interest (411) within the image,
wherein the step of taking a plurality of section lines (412) is then done only within said region of interest.

8. The method of any one of claims 3 to 7, wherein after the step of selecting at least one distance class, the distance class(es) selected are accepted as a region of interest (411) and the method is repeated for said region of interest starting from one of the step of taking a plurality of section lines (412) within said region of interest or a subsequent step.
